(19) Europäisches Patentamt
European Patent Office
Office européen des brevets

(11) Publication number: 0 232 569 B1

(12) EUROPEAN PATENT SPECIFICATION

(45) Date of publication of the patent specification: 14.03.90

(51) Int. Cl.⁴: C07D 217/20 // A61K31/47

(21) Application number: 86300241.6

(22) Date of filing: 15.01.86

(54) 1-Benzyl-2-(N-substituted)-carbamoyl-tetrahydroisoquinolines and method for the preparation thereof.

(43) Date of publication of application: 19.08.87 Bulletin 87/34

(45) Publication of the grant of the patent: 14.03.90 Bulletin 90/11

(84) Designated Contracting States: BE CH DE FR GB IT LI NL

(56) References cited:
CHEMICAL ABSTRACTS, vol. 85, 1976, page 14, abstract no. 153745b, Columbus, Ohio, US; H. SHEPPARD et al.: "The dopamine-sensitive adenylate cyclase of the rat caudate nucleus. II. A comparison with the isoproterenol-sensitive (beta) adenylate cyclase of the rat erythrocyte for inhibition or stimulation by tetrahydroisoquinolines", & MOL. PHARMACOL. 1976, 12(5), 854-61
CHEMICAL ABSTRACTS, vol. 96, 1982, pages 53,54, abstract no. 115772c, Columbus, Ohio, US; S.B. LEICHTER et al.: "Effects of chlorpropamide and tolbutamide on adenylate cyclase activity in rat heart and liver", & BIOCHEM. PHARMACOL. 1981, 30(20), 2861-3f the rat erythrocyte for inhibition or stimulation by tetrahydroisoquinolines", & MOL. PHARMACOL. 1976, 12(5), 854-61

(73) Proprietor: T P O "PHARMACHIM", Iliensko Chaussée 16, Sofia(BG)

(72) Inventor: Ivanov, Chavdar Borissov, Komplex Mladost-1 Bl.46-5, Sofia(BG)
Inventor: Mondeshka, Donka Minkova, Rakovski Street 125, Sofia(BG)
Inventor: Berova, Nikolina Dimitrova, Komplex Krasno Selo Bl.194, Sofia(BG)
Inventor: Rakovska, Rossitza Stefanova, Boul. N. Vaptzarov Bl.37, Sofia(BG)
Inventor: Panova, Yordanka Tzolova, D. Debelyanov Street 12, Sofia(BG)
Inventor: Markov, Marko Tzonev, Komplex Mladost-4 Bl.415-B, Sofia(BG)
Inventor: Ovcharov, Radi Georgiev, Bacho Kiro St. 33, Sofia(BG)
Inventor: Usunov, Petko Dimitrov, 3, 31 January, Sofia(BG)
Inventor: Zabunova, Orhideya Boncheva, Komplex Mladost-2 Bl.215, Sofia(BG)

(74) Representative: Silverman, Warren et al, HASELTINE LAKE & CO. Hazlitt House 28 Southampton Buildings Chancery Lane, London WC2A 1AT(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 232 569 B1

ACTORUM AG

## Description

This invention relates to novel 1-benzyl-2-(N-substituted)-carbamoyl-tetrahydroisoquinolines which have been found to be inhibitors of beta-adenylatecyclase and to a method for the preparation thereof.

It is known that beta-adenylatecylase is an enzyme which gives rise to the production of intraocular fluid and the inhibitors of this enzyme can be potential antiglaucoma agents (see Nathanson, Proc. Narh.Acad.Sci. U.S.A. 1980, 87, 7420-4).

It is an object of this invention to provide inhibitors of beta-adenylatecyclase and a method for the production thereof.

According to one aspect of the invention, there are provided 1-benzyl-2-(N-substituted)-carbamoyl-tetrahydroisoquinolines having the general formula:

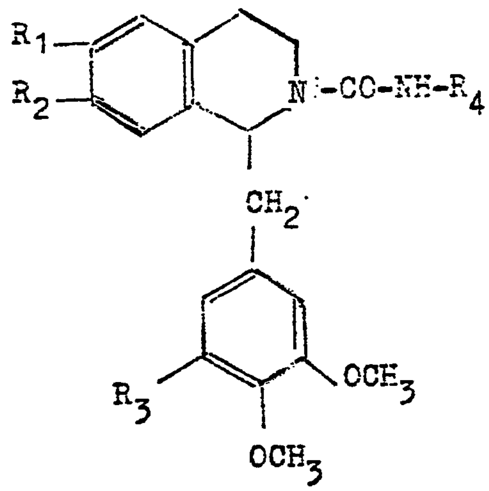

I

in which $R_1$ and $R_2$ are independently hydroxyl or methoxyl groups, $R_3$ is hydrogen or a methoxyl group and $R_4$ is a methyl or chloroethyl group.

According to a second aspect of the invention, there is provided a method for the production of compounds of the general formula I which comprises reacting a tetrahydroisoquinoline of the formula:

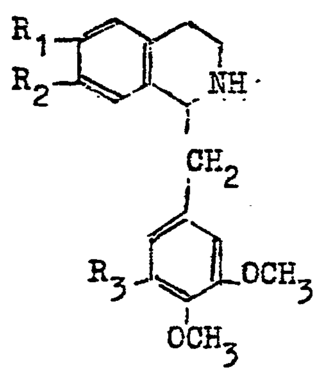

II

in which $R_1$, $R_2$ and $R_3$ have the aforementioned meanings, in equimolar amount with an isocyanate of the general formula:

$O{=}C{=}N{-}R_4$ III

in which $R_4$ has the aforesaid meaning, reaction being carried out in an inert organic solvent at a temperature in the range of from 20°C to the boiling point of the solvent.

The method of this invention utilises the following reaction:

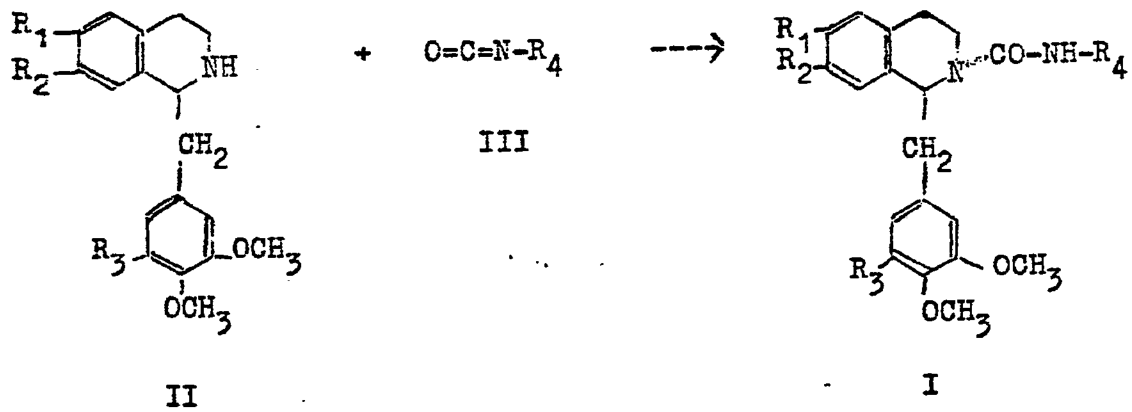

in which $R_1$ to $R_4$ have the aforesaid meanings.

The reaction may be carried out in a chlorinated hydrocarbon medium, for example in chloroform, tetrachloromethane, dichloromethane or methylenedichloride, in an aromatic hydrocarbon medium, for example in benzene or toluene, or in an aliphatic ketone medium, for example in acetone or methylethyl ketone. Particularly preferred solvents are chloroform and acetone. Reaction is carried out at a temperature in the range of from 20°C to a boiling temperature of the organic solvent, preferably from 20°C to 110°C if the boilin point of the solvent should be above 110°C.

The starting tetrahydroisoquinolines of general formula II may be prepared by known methods as described for example in ES-493785/G and by B.J.Stenlake in Eur.J.Med.Chem., 1981, 16(6), 515-24.

The isolation of the carbamoyl derivatives of general formula I may be achieved by filtration of the crystalline precipitate which forms during the reaction or by distillation off of the organic solvent in vacuo. In order to purify the carbamoyl tetrahydroisoquinoline, the crude product is contacted with dilute hydrochloric acid and then recrystallised from an organic solvent, for example an aliphatic alcohol such as methanol, ethanol or isopropanol, an aliphatic ketone such as acetone or an ester of an organic acid, such as ethyl acetate.

The starting isocyanate used will be either methylisocyanate or 2-chloroethylisocyanate.

The compounds prepared by the aforesaid method according to the invention have been shown to have the indicated structure by elemental analysis data and IR and NMR-spectroscopy.

While 1-benzyl and 1-phenyl-N-(dialkylaminoalkyl)-carbamoyl tetrahydroisoquinolines are known to have antiarhythmic and bactericidal activity (see for example DE-A-2435168) and 1-benzyl-N-alkylcarbamoyl tetrahydroisoquinolines are known from JP-73-34180 to have anti-epileptic activity, compounds of such type have not hitherto been proposed for use in the inhibition of beta-adenylatecyclase. It has now been found that 1-benzyl-2-(N-substituted)-carbamoyl tetrahydroisoquinolines of the general formula I possess an inhibiting effect with respect to beta-idenylatecyclase activity in rat brain and lung tissue. This affect is most pronounced in the case of the compound s-(+)-(3,4,5-trimethoxybenzyl)-2-[N-(2-chloroethyl)-carbamoyl]-6,7-hydroxy-1,2,3,4-tetrahydroisoquinoline whose activity as an adenylatecyclase inhibitor has been found to surpass that of known adenylatecyclase inhibitors which according to P.Usunov et al, Adr., Cycl., Nucl.Res., 1972, I, 435-53 are typically being phenothiazines, butyrophenones or propanol. Chemical studies carried out utilising the aforementioned preferred compound according to the invention and whose results appear in the following table show that this tetrahydroisoquinoline derivative gives rise to a well expressed and statistically significant decrease in the intraocular pressure on non-narcotisized rabbits when administered locally in the form of an 0.5% solution. The accompanying drawing shows how the desired effect appears as early as the 30th minute, with a maximum at that the 120th minute and the effect remains for a long time, not returning to the initial level over the entire six hour period during which the effect was monitored.

Table

| Inhibiting of beta-adenylatecyclase by s-(+)-1-(3,4,5-trimethoxybenzyl)-2-[N-(2-chloroethyl)-carbamoyl]-6,7-dihydroxy-1,2,3,4-tetrahydroisoquinoline | | |
|---|---|---|
| Tissue | Ia(molar conc.) | Adenylatecyclase activity M/mg/min |
| 1 | 2 | 3 |
| Brain | – | 284+66 |
| Brain | $1.10^{-5}$ | 26+7 |
| Brain | $1.10^{-6}$ | 73+12 |
| Brain | $1.10^{-7}$ | 104+23 |
| Lung | $1.10^{-5}$ | 11+3 |
| Lung | $1.10^{-6}$ | 28+11 |
| Lung | $1.10^{-7}$ | 66+21 |
| Lung | – | 188+36 |

The following Examples illustrate the invention.

EXAMPLE 1

Preparation of s-(+)-1-(3,4,5-trimethoxybenzyl)-6,7-dihydroxy-2-[N-(2-chloroethyl)-carbamoyl]-1,2,3,4-tetraisoquinoline

3.3 g of s-(-)-1-(3,4,5-trimethoxybenzyl)-6,7-hydroxy-1,2,3,4-tetrahydroisoquinoline were suspended in 66 ml of dry chloroform. 0.7 ml of 2-chloroethylisocyanate were then added and the reaction mixture was stirred at ambient temperature for a period of 24 hours and 15 minutes. What was observed was that after addition of the isocyanate, the starting tetrahydroisoquinoline derivative passed into solution and after one hour, formation of crystals of the desired carbamoyl derivative could be observed. At the end of the reaction period, the crystalline precipitate produced was filtered off, washed with chloroform and then suspended in 120 ml of 5% by weight hydrochloric acid solution, while carrying out stirring continuously for 30 minutes. The precipitate present was filtered off. This operation was repeated 5 times. The thus treated product was then washed with water to neutral reaction and was dried at a temperature of 60°C. 2.9 g of the target compound were then obtained, which compound was recrystallised from ethylacetate-methanol. The recrystallisation was carried out at ambient temperature over a period of 24 hours. The crystals obtained were filtered, washed with ethyl acetate and dried at 60°C. 1.3 g of the target compound were thus obtained, this compound having a melting point of 152-154° C and $[\alpha]_D^{22}$ = + 39.13 (methanol, c = 0.55).

| Elemental analysis: for $C_{22}H_{27}N_2O_6Cl$ | | | | |
|---|---|---|---|---|
| Calculated %: | C 58.60 | H 6.03 | N 6.21 | Cl 7.86 |
| Found %: | C 58.20 | H 6.10 | N 6.42 | Cl 7.56 |

| NMR-spectrum (DMSO) | | |
|---|---|---|
| | δ (ppm) | |
| Methylene protons in isoquinoline nucleus | 2.60–3.85 | (m, 8H) |
| $N-CH_2-CH_2-Cl$ | 3.40 | (d, 2H, J=6 Hz) |
| $Ar-CH_2$ | 3.53 | (s, 3H) |
| | 3.62 | (s, 6H) |
| $CH_3O$ | 5.05 | (t, 1H, J=6 Hz) |
| $H_1$ | 6.26 | (s, 2H) |
| | 6.37 | (s, 1H) |
| | 6.45 | (s, 1H) |
| four aromatic protons | 8.48 | (s, 1H) |
| OH groups | 8.59 | (s, 1H) |

EXAMPLE 2

Preparation of s-(+)-2-(3,4,5-trimethoxybenzyl)-2-(N-methylcarbamoyl)-6,7-dihydroxy-1,2,3,4-tetrahydroisoquinoline

1 g of s-(-)-1-(3,4,5-trimethoxybenzyl-(-2-)-N-methylcarbamoyl)-6,7-dihydroxy-1,2,3,4-tetrahydroisoquinoline was suspended in 40 ml of acetone. 0.16 ml of methylisocyanate was added and the reaction mixture obtained was boiled for 10 hours. After allowing the reaction mixture to stand overnight, crystals formed and were filtered off, washed with acetone and dried at 60°C. After recrystallising these crystals twice from acetone-methanol (1:1 by volume), 0.3 g of the target compound were obtained. This compound melted at 195-196°C. $[\alpha]_D^{22} = +34.18$ (MeOH, c = 0.55)

| Elemental analysis: for $C_{21}H_{26}O_6N_2$ | | | |
|---|---|---|---|
| Calculated %: | C 62.68 | H 6.50 | N 6.96 |
| Found %: | C 62.38 | H 6.80 | N 6.56 |
| Infrared (nujol) 1690, 3420 $cm^{-1}$ | | | |

| NMR-spectrum (DMSO) | | |
|---|---|---|
| | δ (ppm) | |
| $CH_3NH$ | 2.40 | (q, 3H, J=5 Hz) |
| methylene protons with $C_3$ and $C_4$ | 2.36–2.54 | (m, 4H) |
| $Ar-CH_2$ | 3.22 | (q, 2H, J=6.58 Hz) |
| three $CH_3O$ | 3.50 | (s, 3H) |
| | 3.58 | (s, 6H) |
| $C_1$ proton | 5.02 | (t, 1H, J=6.5 Hz) |
| -CONH | 5.96 | (q, 1H, J=4 Hz) |
| aromatic protons | 6.22 | (s, 2H) |
| | 6.29 | (s, 1H) |
| | 6.35 | (s, 1H) |
| OH groups | 8.32 | (s, 1H) |
| | 8.45 | (s, 1H) |

EXAMPLE 3

Preparation of 1-(3,4-dimethoxybenzyl)-2-[N-(2-chorethyl)-carbamoyl-6,7-dimethoxy-1,2,3,4-tetrahydroisoquinoline

2.86 g of tetrahydropapaverine were dissolved in 15 ml of chloroform. 0.7 ml of chloroethylisocyanate were added and the reaction mixture obtained was stirred at ambient temperature for 24 hours. The chloroform present was then distilled off to dryness under vacuum. The oily residue thus obtained was suspended in diethyl ether and was left to stand at a temperature of about 0°C for 2 days. A solid amorphous product was obtained which was filtered, washed several times with ether and dried at ambient temperature. The target compound was obtained with melting point 73-76°C in a yield of 1 g.

| Elemental analysis: for $C_{23}H_{28}N_2O_5Cl$ | | | | |
|---|---|---|---|---|
| Calculated %: | C 61.67 | H 6.29 | N 6.25 | Cl 7.91 |
| Found %: | C 61.91 | H 6.35 | N 6.52 | Cl 8.10 |
| Infrared (nujol): 1650, 3340 $cm^{-1}$ | | | | |

| NMR ($CDCl_3$) | | |
|---|---|---|
| | δ (ppm) | |
| Methylene proton with $C_3$ and $C_4$ | 2.60–4.24 | (m, 8H) |
| Methylene proton from $N-CH_2-CH_2-Cl$ | 4.38–4.60 | (m, 8H) |
| $Ar-CH_2$ | 2.96 | (q, 2H, J=6.5 Hz) |
| $C_1$ proton | 4.95 | (t, 1H, J=6.5 Hz) |
| four–$CH_3O$ | 3.82 | (s, 6H) |
| | 3.96 | (s, 6H) |
| –CO–NH | 6.00 | (t, 1H, J=4.58 Hz) |
| Aromatic protons | 6.18–7.20 | (m, 5H) |

EXAMPLE 4

Influence of s-(+)-1-(3,4,5-trimethoxybenzyl)-2-[N-(2-chloroethyl)-carbamoyl]-6,7-dihydroxy-1,2,3,4-tetrahydroisoquinoline on adenylatecyclase activity in rat brain and lung tissue

Homogenates of rat brain and lung tissue were prepared in a tissue:buffer ratio by volume of 1:10, the buffer being tris-HCl of 0.1 m concentration and pH 7.4. Use was made of the radioisotope method of Krishna, Weiss and Brodie (see J.Pharmacol., Expt.Ther., 1968, 163, 379-381). The standard incubation samples contained, in a final volume of 100 mkl, the following components: buffer, 0.1 S3H-adenosinetriphosphate in a concentration of 1 x $10^{-3}$ M, theophylline in a concentration of 1 x $10^{-3}$ M and 3,5-adenosinemonophosphate in a concentration of 5 x $10^{-4}$M. The operational samples contained in addition the test compound suspended in Tween (Registered Trademark). The samples were incubated for 10 minutes. The newly formed labelled 3,5-adenosinemonophosphate was isolated in columns containing neutral alumina. the alumina was mixed with scintillation liquid and counting of the material present took place by means of a scintillation spectrometer.

EXAMPLE 5

Influence of s-(+)-1-(3,4,5-trimethoxybenzyl)-2-[N-(2-chloroethyl)-carbamoyl]-6,7-dihydroxy-1,2,3,4-tetrahydroisoquinoline on ophtalmotone of non-narcotisized rabbits subject to local administration (two drops intraocularly) with an 0.5% by weight solution.

The intraocular pressure changes were measured by means of the tonemeter of Schiotz before administration and then after 30, 60, 120, 140 and 360 minutes. The data obtained were calculated as mm Hg (133 Pa = 1 mm Hg) according to the table of Leydhecker (see W.Leydhecker, Glaukom, Hrsgb.Th., Axeufeld H., 11 Auflage, 1973, 547-71). It is this data which is plotted in the accompanying drawing.

## Claims

1. 1-benzyl-2-(N-substituted)-carbamoyl-tetrahydroisoquinolines having the general formula:

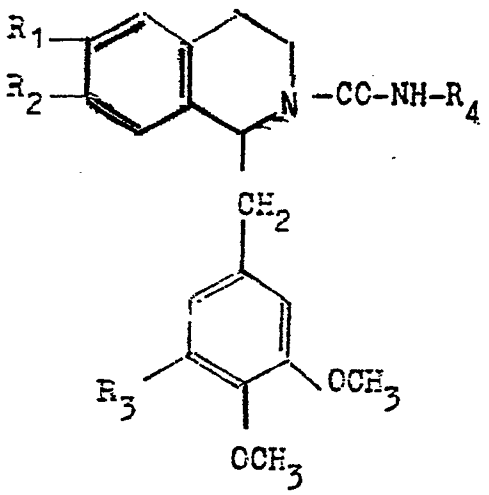

I

in which $R_1$ and $R_2$ are independently hydroxyl or methoxyl groups, $R_3$ is hydrogen or a methoxyl group and $R_4$ is a methyl or chloroethyl group.

2. s-(+)-1-(3,4,5-trimethoxybenzyl)-6,7-dihydroxy-2-[N-(2-chloroethyl)-carbamoyl]-1,2,3,4-tetraisoquinoline

3. s-(+)-2-(3,4,5-trimethoxybenzyl)-2-(N-methylcarbamoyl)-6,7-dihydroxy-1,2,3,4-tetrahydroisoquinoline

4. 1-(3,4-dimethoxybenzyl)-2-[N-(2-chorethyl)-carbamoyl]-6,7-dimethoxy-1,2,3,4-tetrahydroisoquinoline

5. A method for the preparation of 1-benzyl-2-(N-substituted)-carbamoyl-tetrahydroisoquinoline of the general formula I as claimed in claim 1which comprises reacting a tetrahydroisoquinoline of the formula:

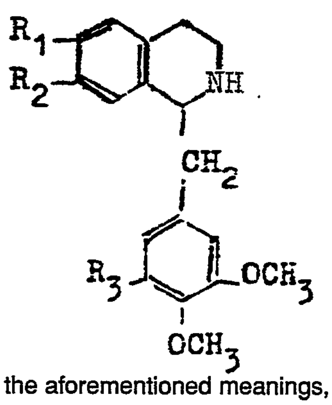

II

in which $R_1$, $R_2$ and $R_3$ have the aforementioned meanings, in equimolar amount with an isocyanate of the general formula:

$O{=}C{=}N{-}R_4$ III

in which $R_4$ has the aforesaid meaning, reaction being carried out in an inert organic solvent at a temperature in the range of from 20°C to the boiling point of the solvent.

6. A method as claimed in claim 5, wherein the tetrahydroisoquinoline used is s-(-)-1-(3,4,5-trimethoxybenzyl)-6,7-dihydroxy-1,2,3,4-tetrahydroisoquinoline or tetrahydropaparavine.

7. A method as claimed in claim 5 or 6, wherein the inert organic solvent is a chlorinated hydrocarbon, an aromatic hydrocarbon or an aliphatic ketone.

8. A method as claimed in claim 7, wherein the solvent is chloroform or acetone.

## Revendications

1. Benzyl-1 (carbamoyl N-substitué)-2 tétrahydroisoquinoléines représentées par la formule générale:

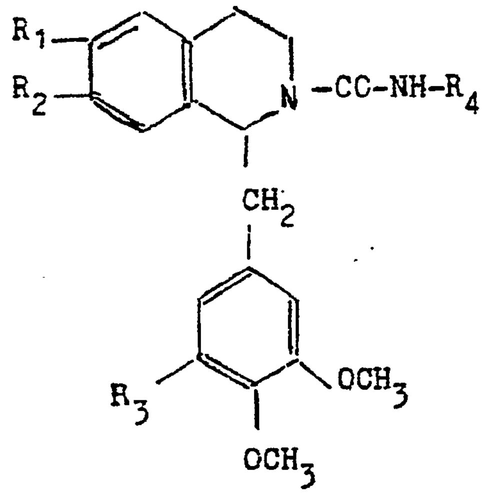

I

dans laquelle:

– $R_1$ et $R_2$ représentent indépendamment des groupes hydroxyle ou méthoxyle;
– $R_3$ représente hydrogène ou un groupe méthoxyle; et
– $R_4$ représente un groupe méthyle ou chloroéthyle.

2. s-(+)-triméthoxy-3,4,5 benzyl)-1 dihydroxy-6,7 [N-(chloro-2 éthyl)-carbamoyl]-2 tétrahydro-1,2,3,4 isoquinoléine.

3. s-(+)-(triméthoxy-3,4,5 benzyl)-2 (N-méthylcarbamoyl)-2 dihydroxy-6,7 tétrahydro-1,2,3,4 isoquinoléine.

4. (Diméthoxy-3,4 benzyl)-1 [N-(chloro-2 éthyl)-carbamoyl]-2 diméthoxy-6,7 tétrahydro-1,2,3,4 isoquinoléine.

5. Procédé de fabrication d'une benzyl-1 (carbamoyl N-substitué)-2 tétrahydroisoquinoléine représentée par la formule générale (I) telle que définie à la revendication 1, qui comprend la réaction d'une tétrahydroisoquinoléine représentée par la formule:

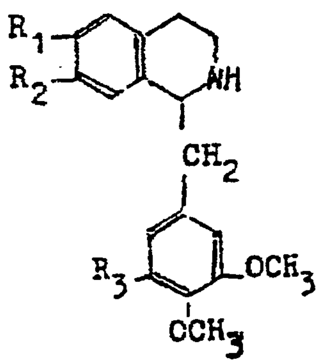

II

dans laquelle $R_1$,$R_2$ et $R_3$ ont les significations mentionnées ci-dessus, en quantité équimolaire avec un isocyanate représenté pa la formule générale:

$O{=}C{=}N{-}R_4$(III)

dans laquelle $R_4$ a la signification précitée, la réaction étant effectuée dans un solvant organique inerte, à une température se situant dans la plage de 20°C au point d'ébullition du solvant.

6. Procédé selon la revendication 5, dans lequel la tétrahydroisoquinoléine utilisée est la s-(–)-(triméthoxy-3,4,5 benzyl)-1 dihydroxy-6,7 tétrahydro-1,2,3,4 isoquinoléine ou la tétrahydropapavérine.

7. Procédé selon la revendication 5 ou 6, dans lequel le solvant organique inerte est un hydrocarbure chloré, un hydrocarbure aromatique ou une cétone aliphatique.

8. Procédé selon la revendication 7, dans lequel le solvant est le chloroforme ou l'acétone.

**Patentansprüche**

1. 1-Benzyl-2-(N-substituierte)-carbamoyltetrahydroisochinoline der allgemeinen Formel

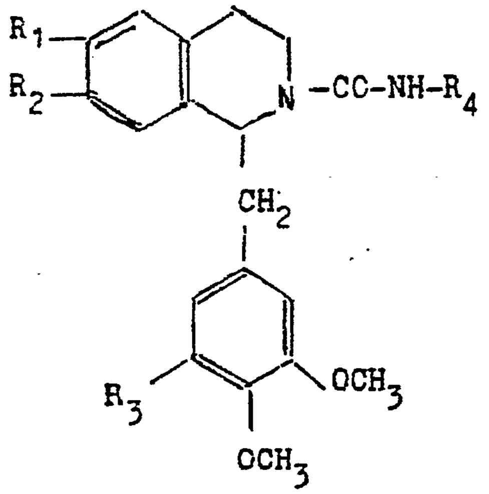

(I)

worin $R_1$ und $R_2$ unabhängig eine Hydroxyl- oder Methoxylgruppe darstellen, $R_3$ Wasserstoff oder eine Methoxylgruppe und $R_4$ eine Methyl- oder Chlorethylgruppe ist.

2. s-(+)-1-(3,4,5-Trimethoxybenzyl)-6,7-dihydroxy-2-[N-(2-chlorethyl)-carbamoyl]-1,2,3,4-tetraisochinolin.

3. s-(+)-2-(3,4,5-Trimethoxybenzyl)-2-(N-methylcarbamoyl)-6,7-dihydroxy-1,2,3,4-tetrahydroisochinolin.

4. 1-(3,4-Dimethoxybenzyl)-2-[N-(2-chlorethyl)-carbamoyl]-6,7-dimethoxy-1,2,3,4-tetrahydroisochinolin.

5. Verfahren zur Herstellung von 1-Benzyl-2-(N-substituiertes)-carbamoyltetrahydroisochinolin der allgemeinen Formel I gemäß Anspruch 1, wobei Tetrahydroisochinolin der Formel

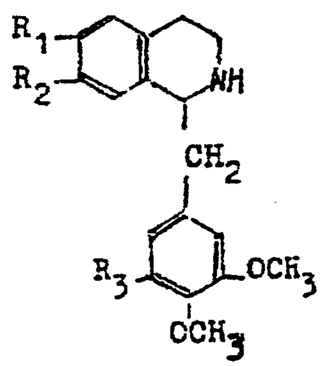

II

worin $R_1$, $R_2$ und $R_3$ die oben angegebenen Bedeutungen haben, in einer äquimolaren Menge mit einem Isocyanat der allgemeinen Formel

$O{=}C{=}N{-}R_4$, III

worin $R_4$ die oben angegebene Bedeutung hat, umgesetzt wird, wobei die Umsetzung in einem inerten organischen Lösungsmittel bei einer Temperatur im Bereich zwischen 20°C und dem Siedepunkt des Lösungsmittels durchgeführt wird.

6. Verfahren nach Anspruch 5, wobei das verwendete Tetrahydroisochinolin s-(–)-1-(3,4,5-Trimethoxybenzyl)-6,7-dihydroxy-1,2,3,4-tetrahydroisochinolin oder Tetrahydropapaverin ist.

7. Verfahren nach Anspruch 5 oder 6, wobei das inerte organische Lösungsmittel ein chlorinierter Kohlenwasserstoff, ein aromatischer Kohlenwasserstoff oder ein aliphatisches Keton ist.

8. Verfahren nach Anspruch 7, wobei das Lösungsmittel Chloroform oder Aceton ist.

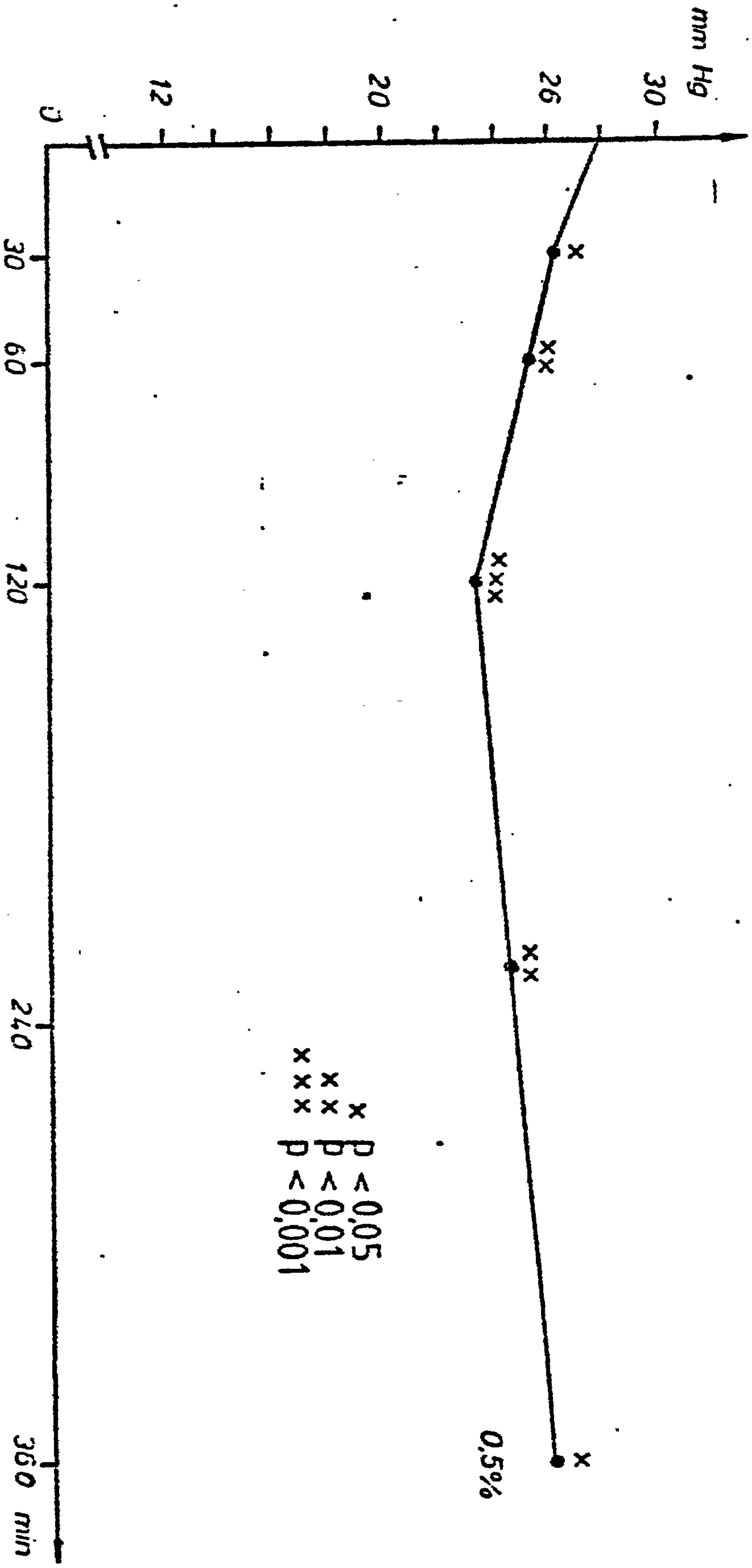
mm Hg
30
26
20
12
0
30
60
120
240
360 min
0,5%
x p < 0,05
x x p < 0,01
x x x p < 0,001